# EUROPEAN PATENT SPECIFICATION

(11) **EP 1 560 848 B1**
(45) Date of publication and mention of the grant of the patent: **09.01.2008**
(21) Application number: 03789032.4
(22) Date of filing: 12.11.2003
(51) Int. Cl.: C07K 14/47, C07K 16/32, A61K 39/00, C12Q 1/68

(54) **PTPRK IMMUNOGENIC PEPTIDE**
PTPRK-IMMUNOGENES PEPTID
PEPTIDE IMMUNOGENE ISSU DE LA PROTEINE PTPRK

(30) Priority: 14.11.2002 IT MI20022412
(43) Date of publication of application: 10.08.2005
(73) Proprietor: ISTITUTO NAZIONALE PER LO STUDIO E LA CURA DEI TUMORI, I-20133 Milano (IT)
(72) Inventor: CASTELLI, Chiara, I-20133 Milano (IT); PARMIANI, Giorgio, I-20133 Milano (IT)
(74) Representative: Banfi, Paolo
(86) International application number: PCT/EP2003/012638
(87) International publication number: WO 2004/043988

(56) References cited:
- WO-A-94/24161
- NOVELLINO LUISA ET AL: "Identification of a mutated receptor-like protein tyrosine phosphatase kappa as a novel, class II HLA-restricted melanoma antigen." JOURNAL OF IMMUNOLOGY (BALTIMORE, MD. : 1950) 15 JUN 2003, vol. 170, no. 12, 15 June 2003 (2003-06-15), pages 6363-6370, XP002291065 ISSN: 0022-1767
- ZHANG Y ET AL: "Cytogenetical Assignment and Physical Mapping of the Human R-PTP-kappa Gene (PTPRK) to the Putative Tumor Suppressor Gene Region 6q22.2-q22.3" GENOMICS, ACADEMIC PRESS, SAN DIEGO, US, vol. 51, no. 2, 15 July 1998 (1998-07-15), pages 309-311, XP004449104 ISSN: 0888-7543
- YANG YOUNG ET AL: "Transforming growth factor-beta-1 inhibits human keratinocyte proliferation by upregulation of a receptor-type tyrosine phosphatase R-PTP-kappa gene expression" BIOCHEMICAL AND BIOPHYSICAL RESEARCH COMMUNICATIONS, vol. 228, no. 3, 1996, pages 807-812, XP002291066 ISSN: 0006-291X
- NAKAMURA MITSUTOSHI ET AL: "Novel tumor suppressor loci on 6q22-23 in primary central nervous system lymphomas." CANCER RESEARCH, vol. 63, no. 4, 15 February 2003 (2003-02-15), pages 737-741, XP002291067 ISSN: 0008-5472

### SEQUENCE LISTING

<110> ISTITUTO NAZIONALE PER LO STUDIO E LA CURA DEI TUMORI
<120> PTPRK IMMUNOGENIC PEPTIDES
<130> 6423MEUR
<160> 20
<170> PatentIn version 3.1
<210> 1
   <211> 16
   <212> PRT
   <213> Homo sapiens
<400> 1
   Pro Tyr Tyr Phe Ala Ala Glu Leu Pro Pro Arg Asn Leu Pro Glu Pro
   1               5                   10                  15
<210> 2
   <211> 48
   <212> DNA
   <213> Homo sapiens
<400> 2
   ccgtattact ttgctgcaga actccccccg agaaacctac ctgagcct 48
<210> 3
   <211> 24
   <212> DNA
   <213> synthetic oligonucleotide
<400> 3
   cgcggatccc gcatggtgtg tctg 24
<210> 4
   <211> 27
   <212> DNA
   <213> synthetic oligonucleotide
<400> 4
   ggaattcctc agctcaggaa tcctgtt 27
<210> 5
   <211> 44
   <212> DNA
   <213> synthetic oligonucleotide
<400> 5
   gactagttct agatcgcgag cggccgccct tttttttttt tttt 44
<210> 6
   <211> 22
   <212> DNA
   <213> synthetic oligonucleotide
<400> 6
   acctcgatta gttctcgagc tt 22
<210> 7
   <211> 24
   <212> DNA
   <213> synthetic oligonucleotide
<400> 7
   attaggacaa ggctggtggg cact 24
<210> 8
   <211> 20
   <212> DNA
   <213> synthetic oligonucleotide
<400> 8
   gtgctcctat cagtgcttat 20
<210> 9
   <211> 18
   <212> DNA
   <213> synthetic oligonucleotide
<400> 9
   gcgtacgcac tgggtttt 18
<210> 10
   <211> 27
   <212> DNA
   <213> synthetic oligonucleotide
<400> 10
   ctgcacccac accgaaccaa gagagaa 27
<210> 11
   <211> 27
   <212> DNA
   <213> synthetic oligonucleotide
<400> 11
   cgcctggaaa tagatgttgt atccttt 27
<210> 12
   <211> 21
   <212> DNA
   <213> synthetic oligonucleotide
<400> 12
   ccgattgtca cccacagtga a 21
<210> 13
   <211> 15
   <212> DNA
   <213> synthetic oligonucleotide
<400> 13
   gggcaggctc aggta 15
<210> 14
   <211> 15
   <212> DNA
   <213> synthetic oligonucleotide
<400> 14
   ctcgggggga gttct 15
<210> 15
   <211> 24
   <212> DNA
   <213> synthetic oligonucleotide
<400> 15
   gggcaggctc aggtaggttt cccg 24
<210> 16
   <211> 18
   <212> DNA
   <213> synthetic oligonucleotide
<400> 16
   ggcgctgcct gcttttgt 18
<210> 17
   <211> 18
   <212> DNA
   <213> synthetic oligonucleotide
<400> 17
   ggaggagcaa tgggtctt 18
<210> 18
   <211> 30
   <212> DNA
   <213> synthetic oligonucleotide
<400> 18
   cctgggatgt agctaaaaaa gatcaaaata 30
<210> 19
   <211> 28
   <212> DNA
   <213> synthetic oligonucleotide
<400> 19
   ccaactaaga tgattccagg tactccaa 28
<210> 20
   <211> 19
   <212> DNA
   <213> synthetic oligonucleotide
<400> 20
   caccctctct ttcagccat 19

### SEQUENCE LISTING

<110> ISTITUTO NAZIONALE PER LO STUDIO E LA CURA DEI TUMORI
<120> PTPRK IMMUNOGENIC PEPTIDES
<130> 6423MEUR
<160> 20
<170> PatentIn version 3.1
<210> 1
   <211> 16
   <212> PRT
   <213> Homo sapiens
<400> 1
   Pro Tyr Tyr Phe Ala Ala Glu Leu Pro Pro Arg Asn Leu Pro Glu Pro
   1               5                   10                  15
<210> 2
   <211> 48
   <212> DNA
   <213> Homo sapiens
<400> 2
   ccgtattact ttgctgcaga actccccccg agaaacctac ctgagcct 48
<210> 3
   <211> 24
   <212> DNA
   <213> Unknown
<220>
   <223> Synthetic oligonucleotide
<400> 3
   cgcggatccc gcatggtgtg tctg 24
<210> 4
   <211> 27
   <212> DNA
   <213> Unknown
<220>
   <223> Synthetic oligonucleotide
<400> 4
   ggaattcctc agctcaggaa tcctgtt 27
<210> 5
   <211> 44
   <212> DNA
   <213> Unknown
<220>
   <223> Synthetic oligonucleotide
<400> 5
   gactagttct agatcgcgag cggccgccct tttttttttt tttt 44
<210> 6
   <211> 22
   <212> DNA
   <213> Unknown
<220>
   <223> Synthetic oligonucleotide
<400> 6
   acctcgatta gttctcgagc tt 22
<210> 7
   <211> 24
   <212> DNA
   <213> Unknown
<220>
   <223> Synthetic oligonucleotide
<400> 7
   attaggacaa ggctggtggg cact 24
<210> 8
   <211> 20
   <212> DNA
   <213> Unknown
<220>
   <223> synthetic oligonucleotide
<400> 8
   gtgctcctat cagtgcttat 20
<210> 9
   <211> 18
   <212> DNA
   <213> Unknown
<220>
   <223> Synthetic oligonucleotide
<400> 9
   gcgtacgcac tgggtttt 18
<210> 10
   <211> 27
   <212> DNA
   <213> Unknown
<220>
   <223> Synthetic oligonucleotide
<400> 10
   ctgcacccac accgaaccaa gagagaa 27
<210> 11
   <211> 27
   <212> DNA
   <213> Unknown
<220>
   <223> Synthetic oligonucleotide
<400> 11
   cgcctggaaa tagatgttgt atccttt 27
<210> 12
   <211> 21
   <212> DNA
   <213> Unknown
<220>
   <223> Synthetic oligonucleotide
<400> 12
   ccgattgtca cccacagtga a 21
<210> 13
   <211> 15
   <212> DNA
   <213> Unknown
<220>
   <223> Synthetic oligonucleotide
<400> 13
   gggcaggctc aggta 15
<210> 14
   <211> 15
   <212> DNA
   <213> Unknown
<220>
   <223> Synthetic oligonucleotide
<400> 14
   ctcgggggga gttct 15
<210> 15
   <211> 24
   <212> DNA
   <213> Unknown
<220>
   <223> Synthetic oligonucleotide
<400> 15
   gggcaggctc aggtaggttt cccg 24
<210> 16
   <211> 18
   <212> DNA
   <213> Unknown
<220>
   <223> Synthetic oligonucleotide
<400> 16
   ggcgctgcct gcttttgt 18
<210> 17
   <211> 18
   <212> DNA
   <213> Unknown
<220>
   <223> Synthetic oligonucleotide
<400> 17
   ggaggagcaa tgggtctt 18
<210> 18
   <211> 30
   <212> DNA
   <213> Unknown
<220>
   <223> Synthetic oligonucleotide
<400> 18
   cctgggatgt agctaaaaaa gatcaaaata 30
<210> 19
   <211> 28
   <212> DNA
   <213> Unknown
<220>
   <223> Synthetic oligonucleotide
<400> 19
   ccaactaaga tgattccagg tactccaa 28
<210> 20
   <211> 19
   <212> DNA
   <213> Unknown
<220>
   <223> Synthetic oligonucleotide
<400> 20
   caccctctct ttcagccat 19

## Description

The present invention relates to immunogenic peptides isolated from the protein PTPRK (Receptor-Like Protein Tyrosine Phosphatase Kappa) and the use thereof in the diagnosis and preventive or therapeutic treatment of tumors. More specifically, the invention provides a novel HLA-Class II restricted epitope recognized by CD4+ T cells and its use in the diagnosis, prevention or immune therapy of patients with melanoma expressing PTPRK.

### BACKGROUND OF THE INVENTION

The identification of tumor associated antigens able to induce a specific anti-tumor T cell response has provided a new immunological approach for the treatment of tumors. The large majority of tumor-associated antigens that have been up to now defined are recognized by HLA-Class I restricted cytotoxic T lymphocytes (CTL), despite the crucial role played by CD4+ T cells which recognize HLA-Class II antigens for the generation and maintenance of an effective immune response in viral infection as well as in cancer. Lack of tumor-specific epitopes able to evoke a T cell-mediated helper response and the self-nature of the majority of HLA-class I restricted tumor-associated antigens constitute the major factors limiting the therapeutic efficacy of vaccination trials in cancer patients. Therefore, the characterization of novel MHC class II-restricted tumor-specific antigens appears of primary importance in order to provide new and more efficient peptide-based vaccines. The mechanisms of class II MHC antigen processing/presentation pathway appear rather complex and not completely understood. Technical difficulties have hampered the discovery of class II HLA-restricted tumor antigens, thus limiting the availability of known tumor-specific helper epitopes for melanoma, the most immunogenic among the human tumors.

PTPRK belongs to the family of the receptor-like plasma membrane-spanning PTP molecules, and it is characterized by the presence in its extracellular portion of fibronectin type III repeats, immunoglobulin and meprin/A5/µ (MAM) domains (30, 31). PTPRK is expressed in normal tissues such as spleen, prostate, ovary, and keratinocyte epidermal cell lines but not in PBL and hematological cell lines (24). Though the precise physiological role of PTPRK is still unclear, its structural features and the ability to mediate homophilic interaction among cells together with the observation that the expression of PTPRK is induced by cell density strongly suggest a crucial role of this protein in the regulation of cell-cell contact formation (30). Moreover, it has been recently demonstrated that in humans PTPRK co-localizes and is associated with β- and γ- catenin at the cell-cell contact area of adjacent cells (25). These findings strongly support the involvement of this protein in negatively regulating the action of tyrosine kinase-induced events at cell junctions, possibly through the dephosphorilation of β- and γ- catenin. These functional roles, and the finding that human PTPRK gene has been mapped to the putative tumor suppressor gene region 6q22.2-q22.3 (31, 32), which is frequently deleted in melanomas (33, 34), overall support the hypothesis that loss of expression as well as mutations occurring in functional domains of the PTPRK protein, could directly affect the phenotype of normal growing cells and play a role in tumor transformation and progression.

### DISCLOSURE OF THE INVENTION

With the aim of identifying HLA-class II melanoma antigens, we studied the CD4+ anti-tumor response in a melanoma patient who experienced a favorable prognosis being disease free 9 years after surgical resection of a lymph node metastasis. This positive clinical evolution was associated with a strong and persistent CD8 mediated anti-tumor response directed against the differentiation antigen gp100 and TRP-2. Since CD4 T cells are known to be essential for the generation and maintenance of CD8 T cells, we investigated the possibility that the patient had developed also a tumor-specific CD4-mediated response.

T lymphocytes obtained from tumor infiltrated lymph nodes were repeatedly stimulated in vitro with the autologous tumor and T cell clones were generated by limiting dilution. CD4+ T cells clones recognizing the autologous tumor in an HLA-DR restricted fashion were obtained, characterized in vitro for their fine specificity and used as cellular probe in a genetic approach aimed at defining the molecular nature of the recognized antigen. The screening of a cDNA expression library constructed using as template the RNA of the autologous melanoma led to the identification of the tyrosine phosphates receptor K gene (R-PTP-K) as encoding the antigen recognized by the CD4+ melanoma specific clones. The R-PTP-K mRNA cloned by melanoma cells contains a non-conservative Gly→Arg mutation in the fourth fibronectin III-like domain of the protein. This amino acid change generates a T cells epitope presented by the HLA-DRβ1*1001 that is recognized by the CD4 T cell clone used to screen the tumor cDNA library and by all the 5 different clones isolated from the tumor infiltrated lymph nodes of the same patient. The antigenic epitope was identified in the region 667-682 of PTPRK_{Gly671→Arg} and it has sequence PYYFAAELPPRNLPEP (SEQ ID N. 1).

A first aspect of the invention is directed to the immungenic peptide of SEQ ID N. 1 and the use thereof in the generation of antibodies and/or T helper or cytotoxic cells, more generally in the induction of a tumor-specific immune response, for diagnostic or therapeutical applications, in particular for the diagnosis, prevention or immune therapy of tumors expressing PTPRK_{Gly677→Arg}.

The peptide of the invention may be prepared following different procedures. For example, it can be synthesized in solution or solid phase according to conventional techniques, or using an automatic synthesizer. The theory and practice of peptide synthesis are known to any skilled person, see for example Stewart and Young (1984) Solid Phase Peptide Synthesis, 2nd ed., Pierce Chemical Co.; Tam et al., J. Am. Chem. Soc., (1983) 105:6442; Merrifield, The Peptides, Gross and Meinenhofer eds. Academic Press (1979) New York, pp. 1-284, herein incorporated by reference.

For use in therapy, the peptide will be suitably formulated together with pharmaceutically acceptable excipients. Suitable formulations for the preventive or therapeutical treatment can be administered orally or parenterally, preferably subcutaneously or intramuscularly, and they will contain an effective amount of the peptide. Said amount should be able to elicit a humoral or cell-mediated immune response directed against the tumor and will vary depending on the general conditions of the patient, the progression of the disease and other factors.

Preferably, the peptide of the invention is administered in the form of a vaccine either for preventive treatment of melanoma-susceptible individuals or for the therapeutic treatment of melanoma patients. The vaccine can be administered according to a single- or multiple- dosage scheme, at suitable doses and at different time intervals so as to maintain or enhance the immune response. The peptide immunogenicity can be increased by cross-linking or coupling with immunogenic carriers, or by use of suitable adjuvants. Furthermore, the peptide may be conjugated with lipids, glucoside residues or other peptides in order to increase bioavailability or the affinity to HLA molecules.

In a further embodiment, the invention provides polyclonal or monoclonal antibodies, fragments or derivatives thereof such as Fab, Fv or scFv, able to recognize and bind the peptide SEQ ID N. 1. The isolated antibodies can be used in tumor immune therapy or in immune diagnostic techniques for the definition of tumors expressing PTPRK_{Gly677→Arg}.

In a yet further embodiment, the invention provides isolated CD4+ T cells specifically recognizing a tumor expressing PTPRK_{Gly677→Arg} and the use thereof for inducing a cell-mediate immune response against such tumor. These cells can be isolated from PBMC obtained from the patient to be subjected to the treatment, and they can be activated in vitro with the peptide SEQ ID N. 1, optionally in the presence of cytokines, or using cells carrying the peptide in association with HLA-Class II molecules, such as APC (antigen presenting cells) expressing the allele HLA-DR β1*1001 loaded with the peptide, APCs can be genetically modified, e.g, by transfection with a viral or retroviral vector, so as to express the specific allele HLA or the peptide or a precursor thereof. Modified HLA cells can be used to activate T cells either in vitro or in vivo. In vitro activated T cells can be subsequently reintroduced in the patient to prevent the onset, to arrest the growth or to reduce the amount of tumor cells. Before being reintroduced into the patient, lymphocytes may be purified, for example by means of an affinity column using an antibody directed against CD4 or other markers.

In a further embodiment the invention provides an isolated nucleic acid molecule encoding the epitope of PTPRK_{Gly677→Arg} herein described, preferably the sequence
CCGTATTACTTTGCTGCAGAACTCCCCCCGAGAAACCTACCTGAGCCT (SEQ ID N. 2)
as well as a vector and a host cell including said sequence. DNA molecules containing the peptide-encoding sequence, or a part thereof, and the gene constructs thereof can be used in the vaccination of subjects at risk of developing tumors, particularly melanoma, or cancer patients. DNA immunization can be carried out according to known techniques (Donnelly J.J. et al., 1994, The Immunologist 2:1). The intramuscular administration route is preferred, but also the parenteral and mucosal routes can be used (pnas 1986, 83, 9551; WO90/11092). Moreover, DNA can be adsorbed onto gold particles for the subcutaneous administration with a biolistic apparatus (Johnston, 1992 Nature, 356, 152).

In addition to the above described therapeutic uses, nucleic acid molecules containing the peptide-encoding sequence, or a part thereof, as well as the peptide itself, can be used in the diagnosis of melanoma expressing PTPRK_{Gly677→Arg} for instance by PCR analysis or immunoassays using epitope-specific antibodies. Furthermore, complexes between the peptide SEQ ID N. 1 and HLA-DR β1*1001 cells can be used for monitoring in vitro or ex vivo the immune response of subjects vaccinated with the peptide.

### DESCRIPTION OF THE FIGURES

### Figure 1

### Functional characterization of anti-melanoma T lymphocyte clone

TB515. (A) Sensitivity of ⁵¹Cr-labeled Me15392 and LCL15392 cells to lysis by TB515 clone: ⁵¹Cr-release was measured after 5h. The assay was performed in the absence (■) or in the presence (▼) of the anti-HLA-DR Ab, L243. (B) Cytokines release assay: TB515 clone was incubated overnight with autologous tumor or 15392LCL at 1:1 cell ratio in the presence or absence of the L243. Supernatant was collected, and the content of INF-γ, GM-CSF, TNF-α and IL-2 was evaluated by commercially available ELISA assay.
S.D. ≤ 5 %.

### Figure 2

*Complementary DNA of clone #11 encodes for the TB515 recognized antigen.* (A) CIITA⁺293 were transfected with cDNA#11 in pEAK8.5-Ii alone or together with pcDNA3.1-DRB1*0102 or pcDNA3.1-DRB1*1001, respectively. CIITA⁺293 singly transfected with pcDNA3.1-DRB1*0102 or pcDNA3-DRB1*1001, and CIITA⁺293 co-transfected with recombinant pcDNA3 encoding green fluorescent protein (GFP) and pcDNA3.1-DRB1*1001 were used as negative control. (B) cDNA#11 was subcloned in pcDNA3.1 and cotransfected with pcDNA3.1-DRB1*1001 in CIITA⁺293. Clone TB515 (1 × 10⁵lymphocytes/well) was added to each transfectant and after 24 h, supernatants were collected and the content of IFN-γ evaluated by ELISA. In both panels, Me15392 was used as positive control. Transfectats were all evaluated for the ability to induce IFN-γ release by TB515.

### Figure 3

*(A) PTPRK mRNA expression in Me15392 cells.* 10 µg of poly(A)⁺mRNA obtained from Me15392 were analyzed by Northern blot. Hybridization was performed using an equimolar mixture of three ³²P labeled probes spanning the extracellular, the transmembrane and the intracellular regions of the PTPRK cDNA (See Materials and Methods for details). Lanes were loaded with a comparable amount of mRNA as checked by β-actin housekeeping gene hybridization (data not shown). Samples: Me15392, mRNA obtained from cultured 15392 melanoma cells; PBL, pool of mRNA from 4 healthy donors; A431, mRNA from epidermoid tumor cell line.
*(B) RT-PCR analysis of PTPRK mRNA expression profile.* The region spanning the intracellular phosphatase domain was amplified by the primers F3/Rδthat generate a specific amplification band of 650 bp. The reaction conditions were set within the linear range of DNA amplification. The amount of template was adjusted in order to obtain comparable levels of β-actin, thus allowing direct comparison of amplified PTPRK among the examined samples. As a representative example, evaluation of PTPRK expression by RT-PCR analysis is reported for 5 out of 10 melanoma cell lines examined: 4 metastatic (Me15392, Me335, Me337 and Me349) and one primary (Me366) melanoma cell lines. A PBL pool of 4 healthy donors (PBL), and normal melanocytes (FM2093) are also included.

Picture is representative of 3 independent experiments.

### Figure 4

*Characterization of cDNA #11.* cDNA #11 and related minigenes are represented as boxes aligned to a schematic structure of PTPRK protein. Black square in each minigene indicates the position of an ATG codon in frame with the starting ATG of the full-length gene (GenBank NM_002844). The mutated nucleotide (g→a) occurring at position 2249 is indicated. Minigenes were synthesized by PCR amplification of cDNA #11 using an identical forward primer (F2) coupled with different, nested reverse primers mapping downstream the mutation (EPR1, EPR2, EPR2WT, and EPR3 reverse primers, indicated by the arrows). Minigenes were cloned into expression vector pcDNA3/TOPO and then co-transfected with pcDNA3-DRB1*1001 or pcDNA3-DRB1*0102 into CIITA⁺-293 cells. Clone TB515 (1×10⁵ cells/well) was added to each transfectant, and after 24 h supernatants were evaluated for the content of IFN-γ by ELISA. In the table: +, positive recognition by TB515; -, no recognition by TB515. EP2wt minigene contained the non-mutated (g) nucleotide. Amino acid sequence in the bottom of the figure was deduced from the sequencing of cDNA #11. Abbreviations in the figure: LS, leader sequence; MAM, meprin/A5/R-PTPµ motif; Ig, immunoglobulin-like domain; FNIII, fibronectin type III-like domain; TM, transmembrane; PTP, protein-tyrosine phosphatase domain; R, arginine deriving from the nucleotide g→a mutation.

### Figure 5

*Identification of the TB515 epitope.* (A, B, C) LCL15392 cells (5.0×10³ cells/well) pulsed for 2 h at 37°C with the synthetic peptides in different concentrations were used to stimulate TB515 T lymphocytes. The TB515 (5 × 10³ cells/well) was added, and after 18 h medium was collected and IFN-γ measured by ELISA. Identical results were obtained using LCL3700, sharing only the DRB*1001 with pt15392 (not shown). (D) 5 × 10³ LCL15392 cells were incubated with various concentrations of the competitor peptides for 15 min. Competitor peptides included: PYGFAAELPP**R**NLPEP, modified in position 3, the wild type PYYFAAELPPGNLPEP, and the HLA-A3 binding peptide ILRGSVAHK which was used as negative control. PYYFAAELPPRNLPEP peptide was then added at 100 nM. After 1 hr of additional incubation at 37°C, TB515 (5×10³ cells/well) was added, and after 18 h medium was collected and IFN-γ measured by ELISA. Identical results were obtained using LCL3700 sharing only the DRB*1001 with pt15392. Mutated amino acid is written in bold; substituted amino acids are underlined.

### Figure 6

*Peptide specificity of T lymphocyte clone TB48.* LCL15392 cells (5.0×10³ cells/well) pulsed for 2 h at 37°C with the synthetic peptides in different concentrations were used to stimulate TB48. The TB48 clone (5×10³ cells/well) was added, and after 18 h medium was collected and IFN-γ measured by ELISA. Identical results were obtained using LCL3700, sharing only the DRB*1001 with pt15392.

### Figure 7

*PTPRK epitope specific immunity in PBMCs of pt 15392.* PBMCs of pt15392, obtained during the disease-free period 12 months after surgery, were stimulated in vitro with the mutated PYYFAAELPPRNLPEP peptide. At the end of the third week of culture, the generated T cell lines were evaluated by the ELISPOT assay for the capacity to release IFN-γ in response to peptide or autologous tumor stimulation, in the presence or in the absence of the anti-HLA-DR Ab L243. T cells were incubated with (1) medium, (2) LCL15392, (3) LCL15392 pulsed with 4 µg of PYYFAAELPPRNLPEP, (4) LCL15392 pulsed with 4 µg of PYYFAAELPPRNLPEP and incubated with 10µg/ml of anti- HLA-DR L243 Ab, (5) LCL15392 pulsed with 2 µg of PYYFAAELPPRNLPEP, (6) LCL 15392 pulsed with 2 µg of PYYFAAELPPRNLPEP and incubated with 10µg/ml of anti-HLA DR Ab L243, (7) LCL15392 pulsed with 4 µg of the wild type PYYFAAELPPGNLPEP peptide, (8) autologous Me15392, (9) autologous Me15392 incubated with anti-HLA DR Ab L243.

**Abbreviations:** pt15392, patient 15392; LN, lymph node, gp100, glycoprotein 100; TRP-2, tyrosinase-related protein 2; PTPRK, receptor-like protein tyrosine phosphatase kappa; RT-PCR, reverse transcribed polymerase chain reaction; CIITA, Class II Transactivator; Ii, invariant chain; GFP, green fluorescent protein; LCL, lymphoblastoid EBV-trasformed B cells line, MTS, melanosomal transport signal; MAM, meprin/AS/PTPRµ, motif; E, effector cell; T, target cell; TCR, T cell receptor.

### MATERIALS AND METHODS

**Cell lines.** The clinical course of patient (pt) 15392 (HLA-A*0301, B*40012, B*1402, C*0602, C*8002, DRB1*0102, DRB1*1001), and the in vitro stabilization of the melanoma cell line Me15392 have been already described (16). By cell surface analysis Me15392 cells were shown to be positive for class I HLA and to constitutively express DR, DP, but not DQ class II HLA. LCL15392 and LCL3700 are EBV-trasformed B cell lines obtained from peripheral blood mononuclear cells (PBMCs) of pt15392 and of an healthy donor, respectively. LCL3700 shares with pt15392 the DRB1*1001 only. 293-EBNA cells (wt293) (Invitrogen, CA 9200, USA) and Class II Transactivator⁺ 293-EBNA cells (CIITA⁺293) were maintained in DMEM medium (Euroclone, Europe, TQ4 5ND Devon, UK) with 10% FCS. CIITA⁺293 cells were obtained by trasducing wt293 cells with CIITA-encoding retroviral vector. CIITA⁺293 cells were immunoselected using L243, a mAb specific for HLA-DR alleles.

**Generation of tumor-specific T cell clones**. Tumor infiltrating T lymphocytes (TIL), collected from a lymph node metastatic lesion surgically removed from pt15392 in 1991, were isolated by density-gradient centrifugation. T cells were stimulated twice in vitro with irradiated autologous tumor cells in RPMI medium supplemented with 300 IU/mL of recombinant human IL-2 (EuroCetus, Amsterdam, The Netherlands) and 10 % pooled human AB serum. After 3 weeks of in vitro culture, T cells were cloned by limiting dilution and the clones screened for growth at day 15. The specificity of the growing T cell clones was analyzed in a ⁵¹Cr-release assay; 140 independent clones displayed a TCR-mediated lytic activity against the autologous tumor. 40 clones displayed an HLA-DR restricted recognition of the autologous tumor and were selected for further analysis. To confirm the clonality and to classify the clones with identical TCR specificity, the TCR repertoire of each clone was examined by reverse transcribed (RT) PCR, using a panel of TCR AV and TCRBV specific primers as previously described (17).

**Cytokine release assay.** Lymphocytes were seeded (5×10³ in 50 µL) in 96-well U-bottomed plates with 10⁴/well melanoma cells or with 5×10³ autologous LCL pulsed for 2 h at 37°C with different doses of peptides, in a final volume of 0.2 mL of RPMI 1640 supplemented with 10 % of pooled human serum (PHS). After overnight incubation at 37°C, supernatants were collected and cytokine content evaluated by ELISA (Endogen, Woburn, MA 01801, USA).

**Cytotoxic assay**. Sensitivity of target cells to lysis was evaluated by a standard ⁵¹Cr-release cytotoxic assay at different effector: target (E:T) ratio. ⁵¹Cr-release assay was performed as previously described (16).

**HLA-DRB1*0102** and **DRB1*1001 cloning.** cDNA clones encoding the HLA-DRB1*0102 and DRB1*1001chains of pt15392 were obtained by RT-PCR starting from poly(A)⁺ RNA prepared from 15392LCL. Amplification was performed using primers specific for conserved 5' and 3' regions of DRβ1 chains (forward 5'-CGCGGATCCAGCATGGTGTGTCTG-3'; reverse 5'-GGAATTCCTCAGCTCAGGAATCCTGTT-3'), and the PCR products were cloned into pcDNA3.1/V5-His TOPO vector (Invitrogen).

**Construction and screening of the tumor-derived cDNA library.** Poly(A)⁺ RNA isolated from Me15392 cells using the FASTRACK kit (Invitrogen) was converted into cDNA with the Superscript Choise System (Life Technologies) using an oligo(dT) primer [5'-pGACTAGTTCTAGATCGCGAGCGGCCGCCC(T)₁₅-3'] containing a XbaI site (underlined). The cDNA was ligated to EcoRI-BstxI adaptors (Stratagene), digested with XbaI, and inserted into the XbaI and BstxI sites of the polylinker located at the 3' end of the invariant chain cDNA insert (Ii, amino acids 1-80), in the expression vector pEAK8.5/Ii, thus creating an Ii/tumor-derived fusion cDNA library. E. Coli DH5α cells were transformed by electroporation with the recombinant plasmids and selected with ampicillin (0.1 g/liter). The library was divided into 1400 pools of about 100 cDNA recombinant clones each; pools were growth overnight in LB medium plus ampicillin (0.1 g/liter), and plasmid DNA was extracted using the QIAprep 96 plasmid kit (Qiagen).

CIITA⁺293 cells seeded in flat-bottomed 96 microwells (5×10⁴ cells/well) were co-transfected with 150 ng of one pool of the cDNA library and 150 ng of the plasmid containing either HLA-DRB1 or HLA-DRB10, using lipofectAMINE (Invitrogen). After 24 h, CD4⁺T clone TB515 was added to each microculture at 1×10⁴ cells/well; 24 h later, 100 µL of supernatant were collected, and INF-γ production was measured by ELISA assay.

**Construction and sequencing of minigenes.** cDNA #11 sequencing was performed with primers mapping into the regions of the pEAK8.5/Ii vector flanking the insert (forward 5'-ACCTCGATTAGTTCTCGAGCTT-3', reverse 5'-ATTAGGACAAGGCTGGTGGGCACT-3'). The non-conservative point mutation (g→a) was confirmed either by sequencing both DNA strands of amplified products from reverse-transcribed Me15392 poly(A)⁺RNA (forward primer F2 5'-GTGCTCCTATCAGTGCTTAT-3', reverse primer R2 5'-GCGTACGCACTGGGTTTT-3') or from Me15392 genomic DNA (forward primer 5'-CTGCACCCACACCGAACCAAGAGAGAA-3', reverse primer 5'-CGCCTGGAAATAGATGTTGTATCCTTT-3').

Mini-genes were prepared from cDNA #11 as PCR amplification products of different lengths. All the amplicons were obtained using the same sense primer F2 coupled with four different antisense primers: EPR1 5'-CCGATTGTCACCCACAGTGAA-3', EPR2 5'-GGGCAGGCTCAGGTA-3', EPR3 5'-CTCGGGGGGAGTTCT-3', and EPR2WT 5'-GGGCAGGCTCAGGTAGGTTTCCCG-3'. The latter was used for the preparation of the EP2wt minigene containing the wild type nucleotide (underlined in the EPR2WT primer). PCR products were cloned into pcDNA3.1/V5-His TOPO vector.

The Ii-EP2wt fusion minigene was obtained by excision of EP2wt minigene from TOPO vector with AscI and XbaI enzymes, followed by cloning the insert into the AscI and XbaI sites of pEAK8.5/Ii vector. This reaction gave rise to an in frame fusion construct.

**Northern Blot and RT-PCR analysis of R-PTPK expression**. Poly(A)+ RNAs from Me15392, allogenic melanomas and PBL lines were isolated as described above. Total RNA was isolated by using RNAqueous^{™}-4PCR kit (Ambion, Austin, TX, 78744, USA). For Northern blot experiments, 10 µg of each RNA sample was subjected to electrophoresis in a 1 % formaldehyde agarose gel and transferred to a nylon membrane (Hybond-N+, Amersham Biosciences, Inc. Piscataway, NJ 08855-1327, USA). The probes were labeled with [alpha-³²P]CTP by the random priming method (Amersham Biosciences), and pre-hybridization and hybridization were performed according to the Hybond-N+ paper guidelines. Membranes were washed four times with serially diluted solutions of SSC (from 0.03M to 0.0015M). Probes A and C were obtained by PCR amplification of Me15392 poly(A)⁺ RNA with specific primers. Probe A, specific for the 5' region of the gene (bases 241-1110 of the gene), was synthesized with primers forward F1 (5'-GGCGCTGCCTGCTTTTGT-3') and reverse R1 (5'-GGAGGAGCAATGGGTCTT-3'). Probe C, specific for the region encoding the two intracellular phosphatase domains (bases 2925-4547 of the gene), was derived with primers forward F3 (5'-CTTGGGATGTAGCTAAAAAAGATCAAAATA-3') and reverse STOP (5'-CCAACTAAGATGATTCCAGGTACTCCAA-3'). All the amplification products were sequenced before being used as probes. DNA clone #11 (bases 2084-2751 of the gene) was used as probe B.

The RT-PCR analyses of PTPRK expression-profile in normal and tumors cell lines were performed with the forward primer F3 and with the reverse primer Rδ (5'-CACCCTCTCTTTCAGCCAT-3') under the following conditions: 2' 94°C, 34 cycles consisting of 1' 94°C, 2' 54°C, 3' 72°C, and finally 10' 72°C. Conditions were set in order to obtain linear DNA amplification. The amplified DNAs were loaded on agarose gels, stained with ethidium bromide, and analyzed with a dedicated software (Image Master VDL-CS, Amersham Pharmacia Biosciences). Standard deviations were ≤ 5% on triplicate experiments. The level of expression of each sample was normalized for RNA integrity by taking into account the level of expression of the β-actine gene (reaction conditions: 4' 94°C, 21 cycles consisting of 1' 94°C, 2' 68°C, 2' 72°C, and finally 10' 72°C, corresponding to linear DNA amplification).

**Peptides synthesis**. Peptides were synthesized by conventional solid phase peptide synthesis, using Fmoc for transient NH₂-terminal protection, and characterized by mass spectrometry. All the peptides used were >95% pure (Neosystem, Strasbourg, France). Peptides were dissolved at 5 mg/mL in DMSO, stored at -20°C, and diluted in RPMI medium supplemented with 10 % human serum immediately before use.

**Epitope reconstitution assay.** To analyze peptide recognition, 5 × 10³ LCL1S392 or LCL3700 cells were seeded in 96 microwells in 100 µl of RPMI 1640-10% PHS and then pulsed with different concentrations of the relevant peptide. Peptide loading was allowed to proceed for 2 h at 37°C before effector cells were added to give a final E:T ratio of 1:1. Supernatants were collected after 18 h and IFN-γ content was determined by ELISA (Mabtech AB, Stockholm, Sweden). Competition experiments were performed incubating 5 × 10³ LCL15392 or 5 × 10³ LCL3700 with various concentrations of the competitor peptides for 15 min before the addition of the PYYFAAELPPRNLPEP peptide at 100nM. After 1 hr of additional incubation at 37°C, T cell clone was added at the final ratio of 1:1.

**Generation of PTPRK specific T cells from pt15392 PBMCs.** PBMCs of pt15392 obtained at 12 months after surgery, during the disease-free period of follow up, were stimulated in vitro with PYYFAAELPPRNLPEP peptide as previously described (15). Briefly, PBMCs (2×10⁶/well) were weekly stimulated with autologous peptide-pulsed monocytes. At the end of each stimulation, peptide-specific reactivity was monitored by Elispot assay.

IFNγ-Elispot assay. 96-well nitrocellulose plates (Millititer, Millipore, Bredford, MA) were coated overnight with 50µl/well of 8µg/ml anti-human IFN-γ mAb (Mabtech). Wells were then washed and blocked with Iscove's modified DMEM (BioWhittaker) and 10% human AB-serum for 2 h at 37°C. T cells (2×10³ or 2×10⁴) were mixed with 1.5×10⁴ peptide-pulsed autologous LCL cells and then seeded in the 96 pre-coated wells. T cells incubated with medium alone or with pokeweed mitogen served as negative and positive controls, respectively. After 24 h of incubation at 37°C and 5% CO₂, Elispot was then performed according to manufactory instructions. Briefly, plates were washed six times with PBS + 0.05% Tween-20. Wells were incubated for 2 h at 37°C with 50 µl/well of biotinylated mouse anti-human IFN-γ mAb (Mabtech,) at a concentration of 2.5 µg/ml. After washing four times with PBS, 100µl streptavidine-alkaline phosphatase (150 µg/ml) diluted 1/1000 was added for 2 h at room temperature. After another washing step with PBS, 100µl/well of BCIP/NBT substrate (BioRad, CA, 94547,USA) was added to each well for 10-20 min. Color development was stopped by washing under running tap water. After drying at room temperature, IFNγ secreting T cells were counted using the automated image analysis system Elispot Reader (AID, Strassberg, Germany). Each experiment was performed in triplicate.

### RESULTS

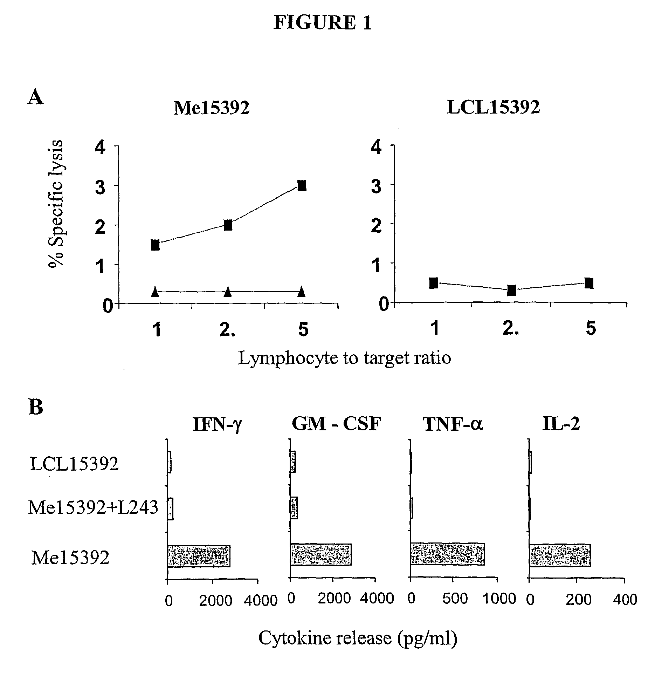

**Tumor-specific CD4⁺T cell clones.** 40 tumor-specific CD4⁺T cell clones (15392CD4⁺T) were obtained by limiting dilution cloning of TIL isolated from a LN metastasis of pt15392, and stimulated in vitro with the autologous tumor for two weeks. TCR analysis by RT-PCR revealed that all clones could be grouped in 5 different subsets according to the TCRAV and TCRBV combination (Table ). Functional studies were performed on a single T cell clone representative of each subset. All the clones were found to display identical functional activity in response to tumor stimulation. Data reported in figure 1 were obtained with clone TB515 and are representative for all the T cell clones tested. Upon stimulation with the autologous melanoma, all the clones exerted an HLA-DR restricted lytic activity against Me15392 cells, since the lysis detected by a ⁵¹Cr release assay, was strongly inhibited in the presence of L243, a mAb directed to a monomorphic determinant of HLA-DR (Fig. 1A). Moreover, upon tumor stimulation the tumor-specific CD4⁺ T cell clones also released a cytokine cocktail compatible with Th1 profile, including INF-γ, GM-CSF, TNF-α and IL-2, but not IL-4 and TGFβ (Fig. 1B).

**Mutated PTPRK gene encodes the TB515 epitope**. Due to its in vitro growth capacity, TB515 was further selected and used to molecularly characterize the melanoma specific antigen. The genetic approach employed for this purpose was developed as an optimization of recent reported methodologies used for the identification of tumor antigens (18-20). The 293-EBNA1 cells stably transfected with CIITA cDNA (CIITA⁺293) (21) were used as recipients for the cDNA tumor library. FACS analysis with anti- HLA-DR Ab revealed a significant increase in the expression of class II HLA on CIITA⁺293 cells (not shown). To force the entrance of the tumor-derived proteins into the class II HLA processing pathway, and thus potentially improving the sensitivity of our screening method, we constructed a chimeric invariant chain (Ii)/tumor cDNA library (22). The cDNA library divided into pools was then transfected into CIITA⁺293 cells together with 100 ng of plasmid containing the DRB1*0102 or DRB1*1001 alleles cloned from the same patient.

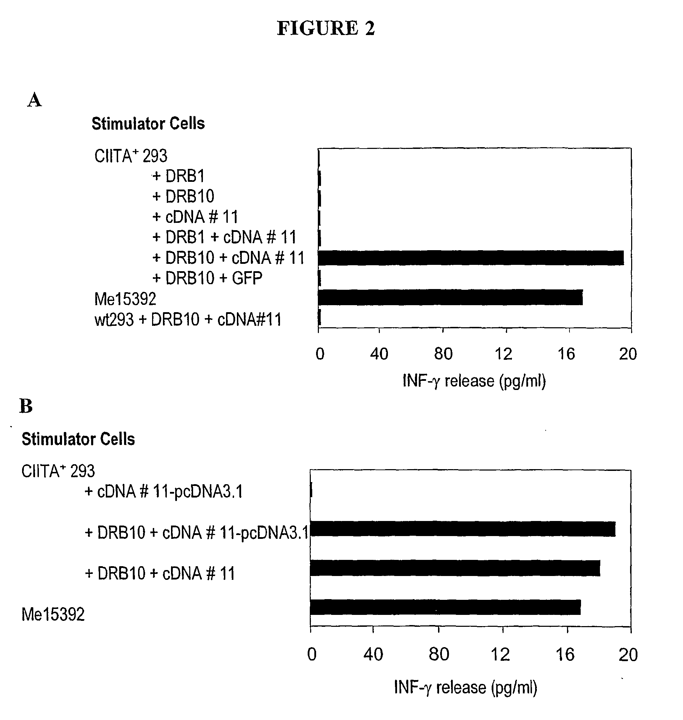

The screening of transfectants led to the isolation of one positive cDNA clone (cDNA #11) that was recognized by TB515 T cells when co-transfected into CIITA⁺293 with DRBI*1001 (CIITA⁺-DRB10⁺293) but not when co-transfected with the DRB1*0102 allele (CIITA⁺-DRB1⁺293) (Fig. 2A). No recognition occurred when cDNA #11 and DRB1*1001 were transfected into 293 not modified by CIITA (wt293), thus further confirming the requirement of an active Class II-HLA processing machinery for TB515 activation.

DNA sequencing analysis indicated that cDNA #11 was homologous to a partial region of the Receptor-Like Protein-Tyrosine Phosphatase Kappa (PTPRK, GenBank NM_002844), a type II tyrosine phosphatase (PTPs), which is one of the five subfamilies of transmembrane receptor-like PTPs (23). Tumor derived cDNA #11 extended from 2084bp to 2751 bp of the published NM_002844 PTPRK cDNA sequence, encompassing the region coding for the fourth C-most extracellular fibronectin III-like domain, the whole transmembrane domain, and the very initial intracellular sequence. The clone presented a non-conservative g→a point mutation at nucleotide 2249 in the fourth fibronectin III-like domain of the gene (NM_002844), that led to a Glycine-to-Arginine substitution (G→R) in the corresponding protein (the GenBank accession number for the sequence of PTPRK cDNA derived from Me15392 is AF533875).

In order to confirm that this amino acid change did not account for a single nucleotide polymorphism, but represented a somatic mutation occurring in tumor tissue, the region encompassing the mutation was amplified and sequenced starting either from genomic DNA or cDNA obtained from both PBL and LCL of pt15392. None of the analyzed samples showed the g→a transition and only the wild type sequence was found (data not shown). Moreover, DNA and cDNA derived from 10 additional melanoma cell lines were similarly analyzed but none of them displayed any mutation at nucleotide 2249.

**Invariant chain sequence is not necessary for the HLA-class II presentation of R-PTPK derived epitope**. Sequence analysis of the recombinant plasmid containing the cDNA clone #11 revealed that the Ii chain translation was shifted in respect to the frame corresponding to the proper translation of PTPRK protein. Therefore, we postulated the existence in the recombinant plasmid of an additional open reading frame directed by an additional internal ATG. By analyzing the sequence of cDNA#11, we found a Kozak-like sequence ((g/a)nnatgg) whose ATG (position 2165 in the GenBank NM_002844 sequence) was in frame with the authentic first starting methionine of the PTPRK gene. To verify the self-sufficient translation capacity of this inner ATG codon, cDNA #11 was excised from pEAK8.5/Ii vector and inserted into the Ii-lacking expression vector pcDNA3.1. The new recombinant plasmid was then evaluated for the capacity to trigger the TB515 activation when transfected into CIITA⁺293 cells together with the DRB *1001 (Fig. 2B). As expected, the cDNA #11 cloned in the absence of the Ii chain induced INF-γ release by TB515 clone in a specific DRB1*1001-restricted fashion. The recognition was as efficient as that of cDNA #11 in pEAK8.5/Ii vector or Me15392 tumor (Fig. 2B) indicating that, in the absence of Ii start codon, cDNA #11 could be translated from its own internal ATG and, moreover, that cDNA #11 could be naturally processed and presented in a HLA-class II pathway per se with no need for an Ii chain mediated intracellular redirection.

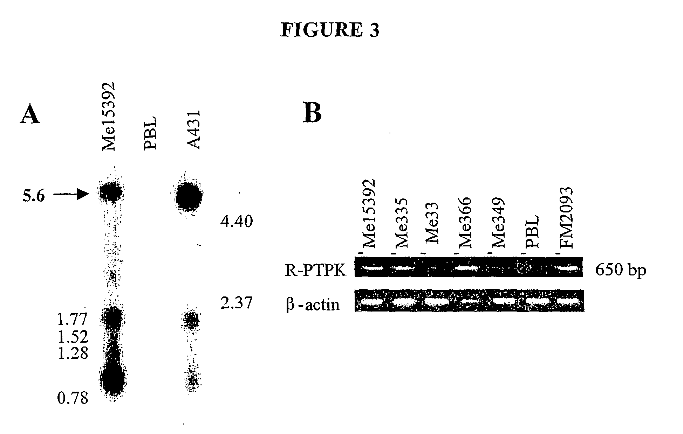

**Expression patterns of R-PTPK mRNA**. To obtain more information about the PTPRK specific transcripts in Me15392, Northern blot analysis was performed (Fig. 3). To encompass the whole PTPRK mRNA, hybridization was carried out using an equimolar mixture of three specific probes which mapped in different regions of PTPRK gene. The examined samples also included PBL poly(A)⁺RNA, as a negative control of PTPRK expression, and transformed epidermal cell line A431, as a positive control of the full-length transcript since this cell line is known to have a high level of PTPRK gene expression (24). Melanocytes could not be analyzed, since no sufficient mRNA was obtained form the in vitro growing cells. As expected, no significant hybridization band was observed in PBL, thus confirming previous data (24), while both A431 and Me15392 poly(A)⁺ RNA gave rise to a rather complex pattern (Fig. 3A). Although additional specific bands around 1800 bp and 1000 bp were clearly detectable, both samples presented an intense signal at about 5600 bp corresponding to the full-length transcript. All together, these results indicate that Me15392 may express a complete PTPRK protein, while the biological significance of the partial mRNA transcripts remains to be addressed.

The expression of PTPRK mRNA in other tumor and non-tumor cells was studied by RT-PCR. RT-PCR amplifications were carried out from total RNA using the primers pair F3/Rδ, mapping in the intracellular, N most phosphatase domain (amplification product length 650 bp). Primers were designed to be highly specific for PTPRK gene. Their specificity was experimentally confirmed, and amplification products obtained from different cell lines were sequenced and showed to correspond only to PTPRK, while no sequences of other R-PTP or PTP genes, even those with high homology to PTPRK, were found. Reaction conditions were set to have linear DNA amplifications, and comparable amount of mRNA was used from each cell lines as evaluated by β-actin amplification. While clearly detectable in melanocytes (FM2093 cell line), PTPRK was differently expressed in 10 melanoma cell lines tested, 5 of which showed only a barely detectable band comparable to that obtained from PBL, that was negative in Northern blot analysis (Fig. 3A). Some representative examples are given in Fig. 3B.

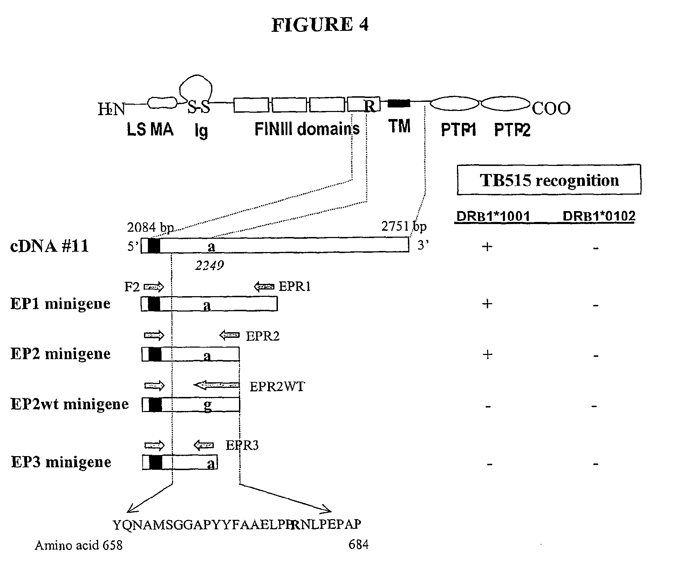

**Identification of PTPRK derived peptide reconstituting the epitope for TB515**. To assess the importance of the G→R mutation for T cell recognition, a wild type (not mutated) version of cDNA #11 was synthesized by PCR reaction and co-transfected with DRB1*1001 cDNA into CIITA⁺293 cells. No recognition of the wild type sequence by the tumor-specific 15392CD4⁺T clones was observed (data not shown), thus suggesting a direct role of the mutated amino acid in the epitope formation. To further sharpen the epitope-coding region of cDNA #11, a series of mini-genes were synthesized by PCR reactions of cDNA #11 using an identical forward primer (F2) coupled with different nested reverse primers mapping downstream the mutation (Fig. 4). The minigenes, which all contained the ATG starting codon of cDNA#11, were cloned into the expression vector pcDNA3/TOPO (Invitrogen), then transfected in CIITA⁺ 293 expressing either DRB1*0102 or DRB1*1001, and finally evaluated for TB515 recognition. The EP2 minigene was the shortest construct being recognized. As expected, the wild type version of the EP2 minigene (EP2WT), obtained using reverse primer EPR2WT bearing the wild type nucleotide (a/g mismatch) was not recognized by TB515 lymphocytes when cloned into the expression vector with or without the invariant chain.

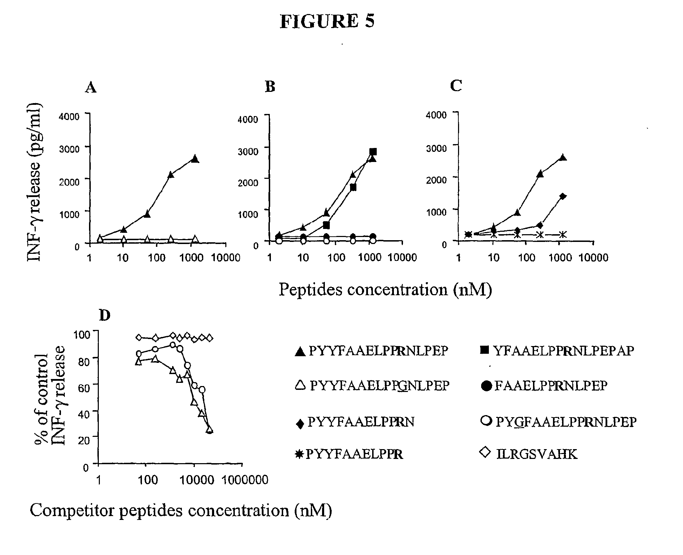

These transfection experiments sharpened the potentially immunogenic amino acid region to a 26 amino acids-long peptide, shown in Fig. 4, that contained the mutation. To identify the TB515 epitope, several hexadecamer overlapping peptides were synthesized that spanned the identified 26 amino acid region. The peptides were evaluated in different doses for their ability to sensitize in vitro autologous LCL cells to recognition by the anti-melanoma TB515 clone. Among the hexadecamer peptides tested, PYYFAAELPPRNLPEP (PTPRK(₆₆₇₋₆₈₂)), which extends from amino acid 667 to 682 of the protein and contains the mutation (Fig. 5, in bold), was the one giving rise to the strongest INF-γ release by TB515 clone. Indeed, titration experiments showed that this peptide could be detected by TB515 cells at a concentration as low as 10 nM, reaching its half-maximum effect at 100 nM (Fig. 5A). The substitution in the immunogenic peptide of the mutated arginine residue with the wild type glycine (Fig. 5A) completely abrogated stimulatory ability, thus definitely showing the relevance of the mutation for T cell mediated recognition. Further experiments with peptides progressively losing an amino acid residue at either the 5' or the 3' end (Fig. 5B and 5C) were aimed at identifying both the minimal epitope core and candidate anchor amino acids. In particular, the omission or the substitution with non relevant amino acids of Y₆₆₉ (Fig. 5B) or L₆₇₉ and N₆₇₈ (Fig. 5C) strongly affected the capacity of the PTPRK(₆₆₇₋₆₈₂) peptide to stimulate TB515 clone, thus suggesting a role for these amino acids in position 3, 11 and 12 in the formation of the HLA-DR10/peptide/TCR complex. The wild type peptide PYYFAAELPPGNLPEP and the PTPRK (667-682) with the Y₆₆₉ substituted by a G were both able to bind the HLA-DR10, since in a competition assay they efficiently inhibited the recognition of the PTPRK(₆₆₇₋₆₈₂) peptide by clone TB515 (Fig. 5D).

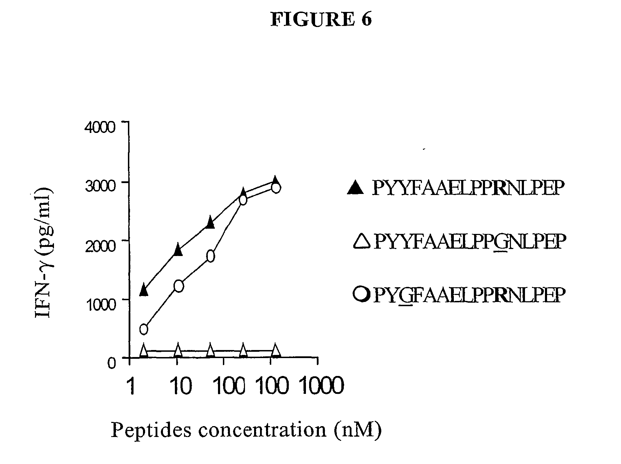

The other 4 T lymphocyte clones described as having different TCR were indeed directed against the same epitope, they did not recognized the wild type peptide and all of them displayed a similar affinity for the mutated peptide as that observed for TB515 (data not shown). Moreover, the PTPRK₍₆₆₇₋₆₈₂₎ peptide bearing a substitution at residue Y₆₆₉ was efficiently recognized by TB48 clone, confirming that Y₆₆₉, although essential for the TB515 mediated recognition, did not crucially influence the HLA-DR10 binding capacity of PTPRK₍₆₆₇₋₆₈₂₎ peptide (Fig. 6).

### Pt15392 developed systemic immunity against the PTPRK derived,

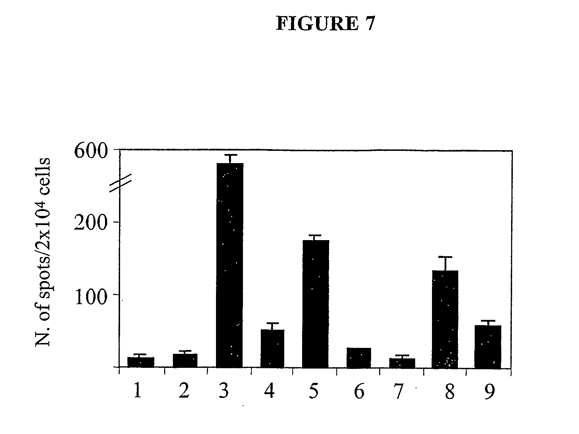

**HLA-DR10 presented epitope.** To establish whether pt15392 also developed a systemic, epitope-specific T cell immunity, PBMCs obtained 12 months after the surgical resection of the lymph node metastasis were cultured in vitro with PYYFAAELPPRNLPEP peptide at 1 µM. After 3 weeks of in vitro stimulation with peptide-pulsed autologous monocytes, the cultured T cells showed a peptide-specific reactivity as detected by Elispot assay, indicating the presence in the peripheral blood of peptide-specific T cells (Fig. 7). These cells were also able to recognize the autologous tumor in an HLA-DR restricted fashion. Conversely, no specific reactivity was detectable in PBMCs obtained from DRB1*1001 positive healthy donors.

**Table.**

| *Functional characterization and TCR expression of tumor-specific CD4⁺T clones.* | | | | | | |
|---|---|---|---|---|---|---|
| | | | **Recognition of the autologous tumor** | | | |
| **^{a}Subset** | **^{b}Clone** | **^{c}TCRAV/TCRBV** | **^{d}Lysis (%)** | | **^{e}IFN-γ release (Pg/ml)** | |
| T1 | TB 515 | 13/21 | 50 | (10) | 1000 | (200) |
| T2 | TB 426 | 3/13 | 74 | (35) | 1200 | (300) |
| T3 | TB 48 | 3/21 | 35 | (15) | 1100 | (200) |
| T4 | TB 674 | 13/19 | 12 | (5) | 800 | (100) |
| T5 | TB 89 | 17/21 | 41 | (10) | 1060 | (200) |

| | | | | | | |
|---|---|---|---|---|---|---|
| ^{a} Forty anti-melanoma 15392CD4⁺T cell clones were generated by limiting dilution of 15392CD4⁺TIL stimulated in vitro with the autologous melanoma for two weeks. The 15392CD4⁺T clones were grouped in 5 distinct subsets (T1-T5) expressing different TCRAV and TCRBV. ^{b} For each subset a tumor-specific clone, further cultured and expanded in vitro, is indicated. ^{c} Analysis of TCR was performed by RT-PCR using a panel of TCR Vα and Vβ specific primers (see Materials and Methods). ^{d} The ability of the indicated clones to lyse the autologous tumor was tested in a 5h ⁵¹Cr-release assay at E:T ratio 30:1. Data are reported as % of lysis. Numbers in parenthesis refer to % of lysis achieved in the presence of the monoclonal anti- HLA-DR L343 mAb used at 10 µg/ml final concentration. ^{e} Cytokine release assay was performed to evaluate the ability of the indicated T cell clones to recognize the autologous tumor (see Material and Methods for details). The amount of IFN-γ released after tumor stimulation in the absence or in the presence (values in parenthesis) of L343 used at 10 µg/ml final concentration is expressed in pg/ml. | | | | | | |

### REFERENCES

1. Parmiani, G., et al., JNCI, 94: 805-818, 2002.
2. Marincola, F.M., et al., Adv. Immunol., 74: 181-273, 2000.
3. Ossendorp, F., et al., Immunol. Lett., 74: 75-79, 2000.
4. Kalams, S.A., and Walker, B.D. J.Exp. Med., 188: 2199-2204, 1998.
5. Overwijk, W.W., et al., Proc. Natl. Acad. Sci. U. S. A., 96: 2982-2987, 1999.
6. Hung, K., et al., J. Exp. Med., 188: 2357-2368, 1998.
7. van Bergen, J., et al., Cancer Res., 60: 6427-6433, 2000.
8. Ossendorp, F., et al., J. Exp. Med., 187: 693-702, 1998.
9. Toes, R.E., et al., Semin. Immunol., 10: 443-448, 1998.
10. Cohen, P.A., et al., Crit. Rev. Immunol., 20: 17-56, 2000.
11. Lennon-Duménil, A. M., et al., Curr. Opin. Immunol., 14: 15-21, 2002.
12. Hiltbold, E.M., Roche, P.A. Curr. Opin. Immunol., 14: 30-35, 2002.
13. Wang, R.F. Trends Immunol., 22: 269-276, 2001.
14. Castelli, C., et al., J. Immunol., 162: 1739-1748, 1999.
15. Castelli, C., et al., Eur. J. Immunol., 28: 1143-1154, 1998.
16. Mazzocchi, A., et al., J. Immunol., 157: 3030-3038, 1996.
17. Castelli, C., et al., Scand. J. Immunol., 35: 487-494, 1992.
18. Chiari, R., et al., Cancer Res., 60: 4855-4863, 2000.
19. Wang, R.F., et al., J. Exp. Med., 189: 1659-1668, 1999.
20. Wang, R.F., et al., Science, 284: 1351-1354, 1999.
21. Sartoris, S., et al., J. Immunol., 161: 814-820, 1998.
22. Sanderson, S., et al., Proc. Natl. Acad. Sci. U. S. A., 92: 7217-7221, 1995.
23. Zhang, Z.Y. Annu. Rev. Pharmacol. Toxicol., 42: 209-234, 2002.
24. Yang, Y., et al., Gene, 186: 77-82, 1997.
25. Fuchs, M., et al., J. Biol. Chem., 271: 16712-16719, 1996.
26. Neel, B.G., and Tonks, N.K. Curr. Opin. Cell. Biol., 9: 193-204, 1997.
27. Hunter, T. Cell, 80: 225-236, 1995.
28. Petrone, A., and Sap, J. Cell. Sci., 113: 2345-2354, 2000.
29. Zondag, G.C., et al., J. Biol. Chem., 270: 14247-14250, 1995.
30. Sap, J., et al., Mol. Cell. Biol., 14: 1-9, 1994.
31. Zhang, Y., et al., Genomics, 51: 309-311, 1998.
32. Guan, X.Y., et al., Cancer Genet. Cytogenet., 134: 65-70, 2002.
33. Maitra, A., et al., Hum. Pathol., 33: 191-197, 2002.
34. McArdle, L., et al., J. Invest. Dermatol., 117: 1255-1260, 2001.
35. Robinson, J.H., and Delvig, A.A. Immunology, 105: 252-262, 2002.
36. Wang, S., et al., J. Immunol., 163: 5820-5826, 1999.
37. Nisini, R., et al., J. Virol., 71: 2241-2251, 1997.
38. Hickling, J.K., et al., Int. Immunol., 2: 435-441, 1990.
39. Topalian, S.L., et al., J. Exp. Med., 183: 1965-1971, 1996.
40. Pieper, R., et al., J. Exp. Med., 189: 757-766, 1999.
41. Yang, S., et al., J. Immunol., 169: 531-539, 2002.
42. Wang, H.Y., et al., J. Exp. Med., 195: 1397-1406, 2002.
43. Wolfel, T., et al., Science, 269: 1281-1284, 1995.
44. Robbins, et al., J. Exp. Med., 183: 1185-1192, 1996.
45. Karanikas, V., et al., Cancer Res., 61: 3718-3724, 2001.
46. Baurain, J.F., et al., J. Immunol., 164: 6057-6066, 2000.

## Claims

1. The PTPRK_{Gly677→Arg} immunogenic peptide of SEQ ID N. 1.

2. A monoclonal or polyclonal antibody, or an active fragment thereof, which selectively binds the peptide of claim 1.

3. An isolated nucleic acid molecule encoding the peptide of claim 1.

4. An expression vector carrying the nucleic acid molecule of claim 3.

5. A host cell containing the vector of claim 4.

6. An isolated CD4+ T lymphocyte able to selectively recognize and bind the peptide SEQ ID N. 1 associated to a HLA-Glass II molecule.

7. A T lymphocyte according to claim 6, which selectively recognizes and binds a peptide/HLA-DR β1*1001 complex.

8. Antigen presenting cells carrying the peptide SEQ ID N. 1 bound to a HLA-DRβ 1*1001 molecule.

9. Pharmaceutical composition containing the peptide SEQ ID N. 1 or a nucleic acid molecule encoding it, in admixture with pharmaceutically acceptable excipients.

10. The pharmaceutical composition of claim 9, in the form of a vaccine.

11. The use of the peptide SEQ ID N. 1, of nucleic acid molecules encoding it, of APCs according to claim 8 or T lymphocytes according to claims 6-7, for the preparation of a medicament for the preventive or therapeutic treatment of cancer.

12. The use claimed in claim 11, for the preventive or therapeutic treatment of melanoma expressing PTPRK_{Gly677→Arg}.

13. The use of peptide SEQ ID N. 1 or of a nucleic acid molecule encoding it for the preparation of a diagnostic composition.

14. The use according to claim 13, wherein said diagnostic composition is utilized in the characterization of melanoma expressing PTPRK_{Gly677→Arg}.

## Patentansprüche

1. PTPRK_{Gly677→Arg} immunogenes Peptid von SEQ ID N. 1.

2. Monoklonaler oder polyklonaler Antikörper oder ein aktives Fragment davon, welcher bzw. welches selektiv an das Peptid gemäß Anspruch 1 bindet.

3. Isoliertes Nukleinsäuremolekül, welches das Peptid gemäß Anspruch 1 codiert.

4. Expressionsvektor, der das Nukleinsäuremolekül gemäß Anspruch 3 trägt.

5. Wirtszelle, die den Vektor gemäß Anspruch 4 enthält.

6. Isolierter CD4+ T-Lymphozyt, der in der Lage ist das Peptid SEQ ID N. 1, welches mit einem HLA-Klasse II Molekül assoziiert ist, selektiv zu erkennen und zu binden.

7. T-Lymphozyt gemäß Anspruch 6, der selektiv einen Peptid/HLA-DR β1*1001-Komplex erkennt und bindet.

8. Antigenpräsentierende Zellen, die das Peptid SEQ ID N. 1 tragen, das an ein HLA-DR β1* 1001-Molekül gebunden ist.

9. Pharmazeutische Zusammensetzung, die das Peptid SEQ ID N. 1 oder ein Nukleinsäuremolekül welches es codiert in Mischung mit pharmazeutisch akzeptablen Hilfsstoffen enthält.

10. Pharmazeutische Zusammensetzung gemäß Anspruch 9, in Form eines Impfstoffs.

11. Verwendung des Peptids SEQ ID N. 1, von Nukleinsäuremolekülen, die es codieren, oder von APCs (antigenpräsentierende Zellen) gemäß Anspruch 8 oder T-Lymphozyten gemäß den Ansprüchen 6-7, zur Herstellung eines Medikaments zur präventiven oder therapeutischen Behandlung von Krebs.

12. Verwendung gemäß Anspruch 11 zur präventiven oder therapeutischen Behandlung von Melanomen, die PTPRKGly_{677→Arg} exprimieren.

13. Verwendung des Peptids SEQ N. 1 oder eines Nucleinsäuremoleküls, welches diese codiert, zur Herstellung einer diagnostischen Zusammensetzung.

14. Verwendung gemäß Anspruch 13, wobei die diagnostische Zusammensetzung bei der Charakterisierung von Melanomen verwendet wird, die PTPRK_{Gly677→Arg} exprimieren.

## Revendications

1. Peptide immunogène PTPRK_{Gly677→Arg} de SEQ ID N° : 1.

2. Anticorps monoclonal ou polyclonal, ou fragment actif de celui-ci, qui se lie sélectivement au peptide de la revendication 1.

3. Molécule d'acide nucléique isolée codant pour le peptide de la revendication 1.

4. Vecteur d'expression portant la molécule d'acide nucléique de la revendication 3.

5. Cellule hôte contenant le vecteur de la revendication 4.

6. Lymphocyte T CD4+ isolé susceptible de reconnaître et se lier sélectivement au peptide de SEQ ID N° : 1 associé à une molécule HLA de classe II.

7. Lymphocyte T selon la revendication 6, qui reconnaît et se lie sélectivement à un complexe peptide/HLA-DR β1*1001.

8. Cellules présentatrices d'antigènes portant le peptide de SEQ ID N° : 1 lié à une molécule HLA-DR β1*1001.

9. Composition pharmaceutique contenant le peptide de SEQ ID N° : 1 ou une molécule d'acide nucléique codant pour celui-ci, en mélange avec des excipients pharmaceutiquement acceptables.

10. Composition pharmaceutique selon la revendication 9, sous la forme d'un vaccin.

11. Utilisation du peptide de SEQ ID N° : 1, de molécules d'acides nucléiques codant pour celui-ci, de CPA selon la revendication 8 ou de lymphocytes T selon les revendications 6 et 7, pour la préparation d'un médicament destiné au traitement préventif ou thérapeutique du cancer.

12. Utilisation selon la revendication 11, pour le traitement préventif ou thérapeutique d'un mélanome exprimant PTPRK_{Gly677→Arg}.

13. Utilisation du peptide de SEQ ID N°: 1 ou d'une molécule d'acide nucléique codant pour celui-ci pour la préparation d'une composition diagnostique.

14. Utilisation selon la revendication 13, dans laquelle ladite composition diagnostique est utilisée dans la caractérisation d'un mélanome exprimant PTPRK_{Gly677→Arg}.
